# EUROPEAN PATENT SPECIFICATION

(11) **EP 3 597 155 B1**
(45) Date of publication and mention of the grant of the patent: **13.11.2024**
(21) Application number: 19186856.1
(22) Date of filing: 17.07.2019
(51) Int. Cl.: A61F 2/88, A61F 2/90, A61F 2/852

(54) **STENT HAVING A STENT BODY AND DETACHABLE ANCHOR PORTION**
STENT MIT EINEM STENTKÖRPER UND LÖSBAREM ANKERABSCHNITT
STENT DOTÉ D'UN CORPS ET D'UNE PARTIE D'ANCRAGE AMOVIBLE

(30) Priority: 17.07.2018 US 201862699090 P
(43) Date of publication of application: 22.01.2020
(73) Proprietor: Cook Medical Technologies LLC, Bloomington, IN 47404 (US)
(72) Inventor: TREACY, Gerard, Limerick (IE); NEILAN, John, Co. Galway (IE)
(74) Representative: Warren, Caroline Elisabeth

(56) References cited:
- WO-A1-00/62708
- WO-A1-2010/030928
- WO-A1-2011/094586
- WO-A2-2007/109621
- DE-A1- 10 105 160
- US-A1- 2006 122 691
- US-A1- 2011 319 977
- US-A1- 2012 310 327
- US-A1- 2017 014 248

## Description

### TECHNICAL FIELD

The present disclosure relates to indwelling medical devices, and more specifically, stents.

### BACKGROUND

Stents may be used to maintain a pathway within a bodily lumen. However, in many bodily areas, such as the esophageal tract, such stents are susceptible to migration from the area in which originally deployed. Such migration is generally undesired because the stent may damage surrounding tissue and may no longer maintain a pathway of the desired lumen. One solution to stent migration is providing a stent with openings that facilitate tissue ingrowth. However, tissue ingrowth is problematic when the stent is designed for temporary deployment. When tissue ingrowth occurs, removal and/or repositioning of the stent is difficult or dangerous.

US-A1-2011/0319977 describes a bioabsorbable implant including an elongated metallic element including more than 50% metal substantially free of rare earth metals, with the elongated metallic element defining at least a portion of the bioabsorbable implant. A hybrid stent may include thin proximal and distal ring segments made from a biostable and radio-opaque material, a larger center ring segment including, e.g., a plurality of rings made of a bioabsorbable magnesium alloy or a helical continuous sinusoid, and filament connectors,

### BRIEF SUMMARY

The invention is set out in claim 1 and preferred features are set out in the dependent claims.

In one aspect, the present disclosure provides a stent with a tubular stent body having a lumen extending therethrough. The stent may further include an anchor portion attached to an end of the tubular stent body, where the lumen continues through the anchor portion, and where the anchor portion includes at least one opening configured to facilitate tissue ingrowth. The anchor portion may be detachable from the tubular stent body while the stent is in a deployed state.

### BRIEF DESCRIPTION OF THE DRAWINGS

The embodiments will be further described in connection with the attached drawings. It is intended that the drawings included as a part of this specification be illustrative of the exemplary embodiments and should in no way be considered as a limitation on the scope of the present disclosure. Indeed, the present disclosure specifically contemplates other embodiments not illustrated but intended to be included in the claims.
FIG. 1 is an illustration showing a stent having a stent body and an anchor portion in accordance with certain aspects of the present disclosure.
FIG. 2 is an illustration showing a magnified view of a proximal end of the stent depicted in FIG. 1.
FIG. 3 is an illustration showing a stent having two anchor portions attached to two respective sides of a tubular stent body in accordance with certain aspects of the present disclosure.
FIG. 4 is an illustration showing a stent having an elongated anchor portion attached to an end of a tubular stent body in accordance with certain aspects of the present disclosure.
FIG. 5 is an illustration showing a magnified view of a stent with an anchor portion being directly attached to a crown of a stent body in accordance with certain aspects of the present disclosure.
FIG. 6 is an illustration showing a magnified view of a stent with an anchor portion being secured to a stent body via a cord (such as a ripcord) in accordance with certain aspects of the present disclosure.
FIG. 7 is an illustration showing a proximal end of a stent having a ripcord with a handle in accordance with certain aspects of the present disclosure.
FIG. 8 is an illustration showing a proximal end of a stent having two strands secured respectively to an anchor portion and a stent body along with a ripcord attaching the two strands together in accordance with certain aspects of the present disclosure.
FIG. 9 is an illustration showing an end of a stent body in a collapsed state in accordance with certain aspects of the present disclosure.
FIG. 10 is an illustration showing a stent, where a stent body of the stent is entering a collapsed state such that it can pass through a lumen of an anchor portion in accordance with certain aspects of the present disclosure.
FIG. 11 is an illustration showing certain components of a stent deployment system in accordance with certain aspects of the present disclosure.
FIG. 12 is an illustration showing the assembled stent deployment system of FIG. 11 in a storage configuration in accordance with certain aspects of the present disclosure.
FIG. 13 is an illustration showing an anchor portion of a stent in a default state when in the storage configuration depicted by FIG. 12 in accordance with certain aspects of the present disclosure.
FIG. 14 is an illustration showing a stent body having a bent crown tip in accordance with certain aspects of the present disclosure.
FIG. 15 is an illustration showing the bent crown tip from FIG. 14 being inserted in an opening of an anchor portion in accordance with certain aspects of the present disclosure.
FIG. 16 is an illustration showing the bent crown tip from FIG. 14 being inserted in an opening of an anchor portion and being secured via a pin in accordance with certain aspects of the present disclosure.
FIGS. 17A-17B are illustrations showing certain embodiments of a crown tip having wing in accordance with certain aspects of the present disclosure.
FIG. 18A is an illustration showing an anchor portion having eyelets in accordance with certain aspects of the present disclosure.
FIG. 18B is an illustration showing the anchor portion of FIG. 18A being secured to a stent body in accordance with certain aspects of the present disclosure.

### DETAILED DESCRIPTION

The embodiments illustrated herein can be used in any portion of the body benefiting from a removable or repositionable indwelling medical device, such as a stent, including but not limited to, the gastrointestinal region, esophageal region, duodenum region, biliary region, colonic region, as well as any other bodily region or field, and they are not limited to the sizes or shapes illustrated herein.

Throughout, patients are not limited to being a human being, and indeed animals and others are contemplated. Users contemplated throughout the disclosure may be anyone or thing capable of using the device, including but not limited to a human being (e.g., a medical professional) and machine.

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. The materials, methods, and examples disclosed herein are illustrative only and not intended to be limiting.

The invention is described with reference to the drawings in which like elements are referred to by like numerals. The relationship and functioning of the various elements of this invention are better understood by the following detailed description. However, the embodiments of this invention are not limited to the embodiments illustrated in the drawings. It should be understood that the drawings are not to scale, and in certain instances details have been omitted which are not necessary for an understanding of the present invention, such as conventional fabrication and assembly.

As used in the specification, the terms proximal and distal should be understood as being in the terms of a physician delivering the medical device to a patient. Hence the term "distal" means the portion of the medical device that is farthest from the physician and the term "proximal" means the portion of the medical device that is nearest to the physician.

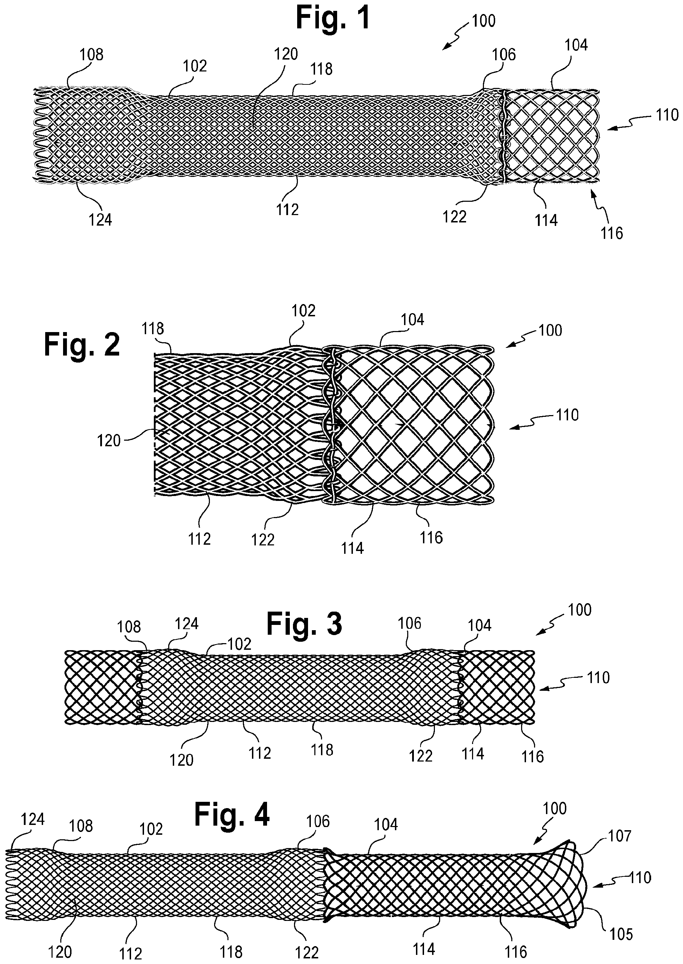

FIG. 1 is an illustration showing a stent 100 with a stent body 102 and an anchor portion 104, and FIG. 2 is a magnified view showing a proximal end of FIG. 1. While such a device can be used in any suitable application (and within any body lumen or other location of a patient body), the stent 100 of FIGS. 1-2 is particularly suitable for use in the GI tract, including biliary and esophageal locations. For example, when treating benign strictures in the esophagus a removable stent is often desired, but existing removable stents do not provide sufficient anchorage and thus tend to migrate. The stent 100 provides a solution to this issue because it includes the stent body 102 (e.g., a covered stent portion) that may be removed after a predetermined period of time in combination with the anchor portion 104 that provides sufficient anchoring. As described in more detail below, the anchor portion 104 may be detachable from the stent body 102 such that the stent body 102 can be removed from a patient body, leaving the anchor portion 104 behind. The anchor portion 104 may remain in the patient body permanently, may be removed separately, and/or may at least partially degrade over time due to its material composition, as described in more detail below.

The stent body 102 may include a generally-tubular shape with a proximal end 106, a distal end 108, and a lumen extending therethrough for providing an interior passage from the proximal end 106 to the distal end 108. The lumen 110 may continue through the anchor portion 104, which may also be generally-tubular in shape. As shown, the anchor portion 104 may be attached to an end of the stent body 102 (e.g., the proximal end, as shown, or the distal end), and in some embodiments an anchor portion 104 may be attached to each of the proximal end 106 and distal end 108 (e.g., as shown in FIG. 3). Including an anchor portion at the proximal end 106 may be particularly advantageous in the GI tract due to the flow-direction of food and other GI contents. To prevent tissue ingrowth through the walls of the stent body 102, the stent body 102 may be covered, coated, or otherwise sealed. For example, the stent body 102 may include a silicone membrane covering, although other materials are contemplated, to seal the exterior surface of the stent body 102. In contrast, the anchor portion 104 may include one or more openings, cells, or other spaces or cavities to promote tissue ingrowth such that the anchor portion 104 becomes sufficiently anchored in an appropriate position within the patient body, thus preventing migration of the stent 100.

The stent body 102 may be any suitable stent structure, such as a self-expanding stent body or a body that expands under pressures or other influence from another device, such as an inflatable balloon at the tip of a balloon catheter. One example of an exemplary stent body is a self-expanding woven or braided stent body, such as a woven stent body formed from a plurality of wires 112 extending in opposite-helical directions around the outer perimeter of the stent body's outer surface. Certain examples of woven stent bodies are described in more detail in U.S. Patent Application Publication No. 2017/0105854, filed October 20, 2015. One particular example is the Evolution^{®} stent commercialized by Cook^{®} Medical.

In other embodiments, the stent body 102 may be formed by a Z-stent design or Gianturco stent design (commercialized by Cook^{®} Medical). In this instance, the stent body 102 may include a series of substantially straight segments or struts interconnected by a series of bent segments or bends. The bent segments may include acute bends or apices. The segments may be arranged in a zigzag configuration where the straight segments are set at angles relative to one another and are connected by the bent segments. The first stent may additionally or alternatively be formed of another stent pattern, such as an annular or helical stent pattern.

Without limitation, the stent segments and/or woven wires mentioned herein may be made from standard medical grade stainless steel or from nitinol or other shape-memory materials, and/or other metals/polymers in either a permanent or bioabsorbable design. It is also contemplated that the stent body 102 may be something other than a self-expanding stent, such as a substantially rigid or obstinate tube formed of a biocompatible material and/or any other suitable device for temporarily or permanently providing support to a body lumen.

The anchor portion 104 may incorporate any of the structures described with respect to the stent body 102 (e.g., the anchor portion 104 may incorporate wires 114 woven in opposite helical directions in certain exemplary embodiments). In some embodiments, the anchor portion 104 may be at least partially formed of a biodegradable and/or bioabsorbable material, such as a material that degrades at a known rate (e.g., hours, days, weeks, or months as required) when the anchor portion 104 is located inside a patient body. For example, without limitation, the frame member(s) (e.g., woven wires or other structure-providing device) may be formed of the materials and techniques discussed in U.S. Patent Application Publication No. 2005/0267560, filed May 23, 2005.

For example, one or more bioabsorbable polymer materials may be used to form the anchor portion 104. Using polymer materials provide the ability to enhance certain characteristics such as flexibility, compliance, and rate of bioabsorption. The properties of the bioabsorbable polymers may differ considerably depending on the nature and amounts of the comonomers, if any, employed and/or the polymerization procedures used in preparing the polymers. Biodegradable polymers that can be used to form the support frame of a medical device (such as the anchor portion 104), or can be coated on a frame member, include a wide variety of materials. Examples of such materials, include but are not limited to, polyesters, polycarbonates, polyanhydrides, poly(amino acids), polyimines, polyphosphazenes and various naturally occurring biomolecular polymers, as well as co-polymers and derivatives thereof. Certain hydrogels, which are cross-linked polymers, can also be made to be biodegradable. These include, but are not necessarily limited to, polyesters, poly(amino acids), copoly(ether-esters), polyalkylenes oxalates, polyamides, poly(iminocarbonates), polyorthoesters, polyoxaesters, polyamidoesters, polyoxaesters containing amido groups, poly(anhydrides), polyphosphazenes, poly-alpha-hydroxy acids, trimethlyene carbonate, poly-beta-hydroxy acids, polyorganophosphazines, polyanhydrides, polyesteramides, polyethylene oxide, polyester-ethers, polyphosphoester, polyphosphoester urethane, cyanoacrylates, poly(trimethylene carbonate), poly(iminocarbonate), polyalkylene oxalates, polyvinylpyrolidone, polyvinyl alcohol, poly-N-(2-hydroxypropyl)-methacrylamide, polyglycols, aliphatic polyesters, poly(orthoesters), poly(ester-amides), polyanhydrides, modified polysaccharides and modified proteins. Some specific examples of bioabsorbable materials include poly(epsilon-caprolactone), poly(dimethyl glycolic acid), poly(hydroxyl butyrate), poly(p-dioxanone), polydioxanone, PEO/PLA, poly(lactide-co-glycolide), poly(hydroxybutyrate-covalerate), poly(glycolic acid-eo-trimethylene carbonate), poly(epsilon-caprolactone-co-p-dioxanone), poly-Lglutamic acid or poly-L-lysine, polylactic acid, polylactide, polyglycolic acid, polyglycolide, poly(D,L-lactic acid), L-polylactic acid, poly(glycolic acid), polyhydroxyvalerate, cellulose, chitin, dextran, fibrin, casein, fibrinogen, starch, collagen, hyaluronic acid, hydroxyethyl starch, and gelatin.

If a metallic bioabsorbable material is used, it may be selected from a first group consisting of: magnesium, titanium, zirconium, niobium, tantalum, zinc and silicon. Mixtures and alloys of metallic bioabsorbable materials including those selected from the group in the preceding sentence are also contemplated. In some embodiments, the metallic bioabsorbable material can be an alloy of materials from the first group and a material selected from a second group consisting of: lithium, sodium, potassium, calcium, iron and manganese. Without being limited to theory, it is believed that the metallic bioabsorbable material from the first group may form a protective oxide coat upon exposure to blood or interstitial fluid. The material from the second group is preferably soluble in blood or interstitial fluid to promote the dissolution of an oxide coat. The bioabsorption rate, physical properties and surface structure of the metallic bioabsorbable material can be adjusted by altering the composition of the alloy. In addition, other metal or non-metal components, such as gold, may be added to alloys or mixtures of metallic bioabsorbable materials. Some preferred metallic bioabsorbable material alloy compositions include lithium-magnesium, sodium-magnesium, and zinctitanium, which can optionally further include gold. The frame members themselves, or any portion of the anchor portion 104, can be made from one or more metallic bioabsorbable materials, and can further include one or more non-metallic bioabsorbable materials, as well as various non-bioabsorbable materials. The bioabsorbable material can be distributed throughout the entirety of the frame member(s), or any localized portion thereof, in various ways. In some embodiments, the frame member(s) can include a bioabsorbable material or a non-bioabsorbable material as a "core" material, which can be at least partially enclosed by other materials. The frame can also have multiple bioabsorbable materials stacked on all or part of the surface of a non-bioabsorbable core material. The frame member(s) can also include a surface area presenting both a bioabsorbable material and a non-bioabsorbable material.

In some embodiments, the anchor portion 104 may have a diameter 116 (e.g., an outer diameter) that is larger than a diameter 118 of the stent body 102. Advantageously, the larger diameter 116 of the anchor portion 104 may ensure that appropriate contact and pressure is experienced between the anchor portion 104 and an inner surface of a body lumen to prevent migration, promote tissue ingrowth, promote bioabsorption, etc. The diameter 116 of the anchor portion 104 may be at least about 5% larger than the diameter 118 of the central portion 120 of the stent body 102, such as about 10% greater. In one non-limiting embodiment for use in a human esophagus, the outer diameter 116 of the anchor portion 104 may be about 25,4 mm (1 ") and the outer diameter 118 of the central portion 120 may be about 19.05mm (0.75"). When the unit inches (") is mentioned in the following, the conversion is 1 "=25,4mm.

Optionally, the stent body 102 may include a proximal flange 122 and/or a distal flange 124 that have diameters greater than the diameter 118 of the central portion. As shown, the diameter of the flanges 122, 124 is approximately equal to the diameter 116 of the anchor portion 104, but this is not required. The flanges 122, 124 may be advantageous to enhance anchorage of the stent body 102 when it is deployed and to accommodate engagement with the anchor portion 104. While not shown in the depicted embodiment, it is contemplated that the flanges 122, 124 may be sized such that their inner diameters are slidably received by the inner diameter of the anchor portion 104, or vice versa. To prevent tissue ingrowth through the outer walls of the flanges 122, 124, the flanges 122, 124 may be covered (as described above), but it is also contemplated that limited tissue ingrowth may be desirable through the flanges 122, 124 in some situations (e.g., to prevent migration of the stent body 102 when the anchor portion 104 degrades).

While the central portion 120 may have a smaller diameter (as shown) than the above-described flanges and/or anchor portions, it is contemplated that the central portion 120 may be more rigid than other portions or otherwise configured to provide a relatively high radial force (e.g., to press back benign tumors), while the flange 122, 124 and/or the anchor portion 104 may be relatively compliant to provide comfort to a patient (particularly when the flange 122, 124 and/or the anchor portion 104 is configured to abut patient tissue (e.g., healthy patient tissue).

As shown in FIGS. 1-2, the anchor portion 104 may be substantially shorter in length than the stent body 102. For example, the anchor portion 104 may have a length that is about 30% of the length of the stent body 102, or less. In one non-limiting embodiment, the length of the anchor portion 104 may be about 0.75" and the length of the stent body 102 may be about 4.5", but other dimensions are contemplated for patients of different sizes, different medical conditions, different target body lumens, etc.

Referring to FIG. 4, it is also contemplated that the anchor portion 104 may be longer than 30% of the length of the stent body 102, particularly when a high-degree of anchoring is desirable. Further, like the stent body 102, the anchor portion 104 may have different diameters along its length, which is illustrated by the flange 105 on the proximal end 107 of the anchor portion 104 of FIG. 4.

As described above, the anchor portion 104 may be detachable from the stent body 102. Preferably, such detachability can occur when the stent 102 is in a deployed state located within the patient body. Advantageously, the anchor portion 104 can be left in the body (or it can disintegrate prior to stent removal) while the stent body 102 can be removed. This detachability may be provided by the attachment mechanism that initially secures the stent body 102 to the anchor portion 104, the material of the stent body 102 and/or the anchor portion 104, by a separate medical instrument, etc.

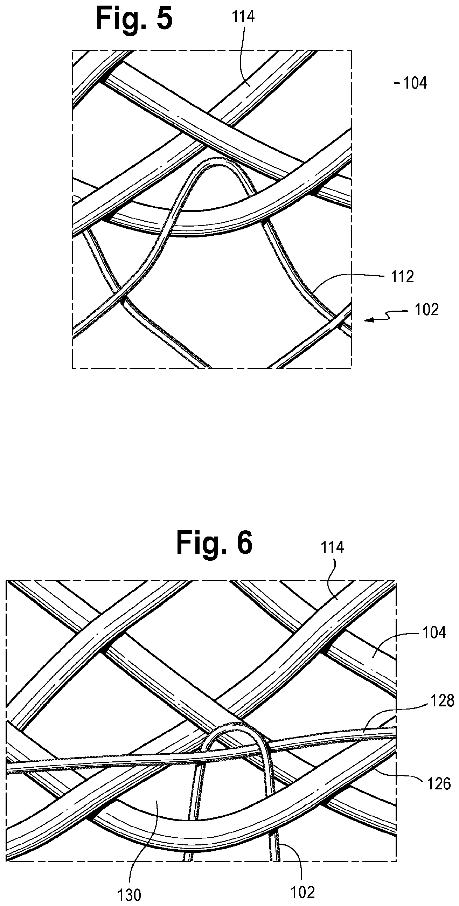

For example, referring to FIG. 5, the stent body 102 and the anchor portion 104 may initially (e.g., prior to and during deployment) be attached and secured to one another by interlocking one or more wires 112 forming stent body 102 with one or more wires 114 forming the anchor portion 104. While the attachment may be relatively secure prior to spending a predetermined amount of time deployed within the body, at least one of the wires 112, 114 (specifically the wires 114 of the anchor portion 104 in certain exemplary embodiments) may weaken over time due to degradation (e.g., by being formed of a bioabsorbable material). After a predetermined amount of time, the wire 114 may be sufficiently degraded such that the wires 114 are easy broken, or are no longer present, when it comes time for removal of the stent body 102. This embodiment may be advantageous since no additional components are needed to attach the two components together, thus simplifying manufacturing and certain medical procedures. While all the wires 114 may be made of the same material, it is also contemplated that the wires 114 that interlock the wires 112 may degrade faster than other wires of the anchor portion 104, which may be advantageous where it is desirable to detach the two elements while the anchor portion 104 is still in-tact enough to provide support to a body lumen.

In other embodiments, including the embodiment depicted in FIG. 6, a separate component, such as a ripcord (or other strand), a clamp, a tie, or any other suitable fastening device may be used to initially secure and attach the stent body 102 with the anchor portion 104. In the depicted embodiment, a cord 128 (which may be a ripcord or any other suitable strand) is used. The cord 128 may extend through openings of one or both of the stent body 102 and the anchor portion 104. Alternatively (and as shown), the cord 128 may extend only through openings in the crown 126 of the stent body 102, which may be advantageous for ease of manufacturing/assembly and/or for consistency in removal of the cord 128. The attachment is accomplished in this embodiment because the cord 128 prevents the protrusions of the crown 126 from being pulled away from the respective openings 130 formed by wires 114 of the anchor portion 104.

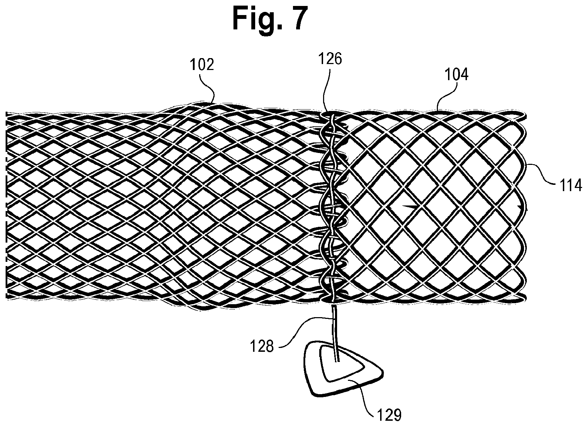

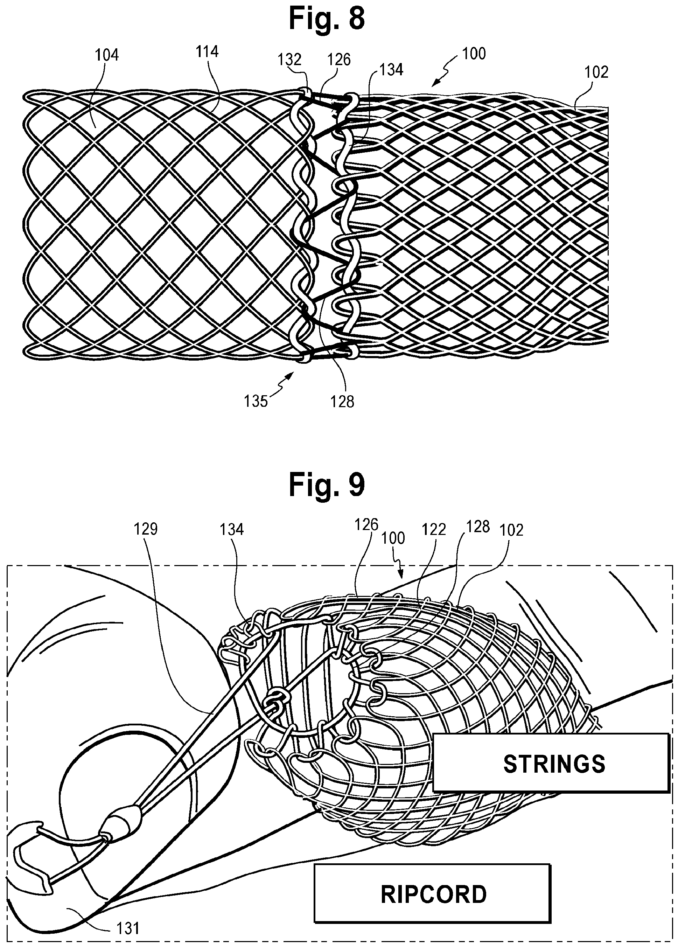

To detach the anchor portion 104 from the stent body 102, the cord 128 can be removed. For example, the cord 128 may be formed of a biodegradable material to allow detachment of the anchor portion 104 from the stent body 102 after a predetermined amount of time in the body (e.g., due to degradation of the cord 128). It is contemplated that the cord 128 may be configured to degrade faster than the material of the anchor portion 104, and thus the anchor portion 104 can be left in the patient body for additional time to provide structure to the body lumen even after the stent body 102 is separated and removed. Additionally or alternatively, the cord 128 can be cut (e.g., with a separate cutting device, such as a knife or scissors) and then pulled out of the patient, leaving the stent body 102 and the anchor portion 104 separated. As shown in FIG. 7, an optional handle 131 (shown in FIG. 7 but only labeled in FIG. 9) may be included to facilitate removal of the cord 128. For example, a separate medical instrument may be configured to find and grasp the handle 131, and then pull on the handle 131 to remove the cord 128.

FIG. 8 shows an embodiment with a cord 128 that engages a first string 132 and a second string 134. In particular, the cord 128 alternates between interlocking with the first string 132 and the second string 134 as it extends around the circumference of the stent 100 at a junction 135 between the stent body 102 and the anchor portion 104. The first string 132 may be secured to the anchor portion 104 (e.g., due to extending through the openings 130 formed by the wires 114), and the second string 134 may similarly be secured to the crown 126 of the stent body 102 (e.g., it may extend through openings formed by the crown 126). It is contemplated that the first string 132 and the second string 134 may be one continuous strand, but they are separate strands in the depicted embodiment. Further, it is contemplated that neither of, or only one of, the first string 132 and the second string 134 may be included. For example, if neither of the first string 132 and the second string 134 is included, the cord 128 extend through the openings 130 and openings of the crown 126 in a zig-zag patter. If only one of the strings is included, the cord 128 may directly secure to one of the anchor portion 104 and the stent body 102 but may be secured to the other by way of a string, for example.

To detach the anchor portion 104 from the stent body 102, at least one of the anchor portion 104, the first string 132, the second string 134, and the cord 128 (or even the stent body 102) may degrade during its time in the body due to including a bioabsorbable material, and/or at least one of those components can be broken (e.g., cut with a cutting device). In one exemplary embodiment, the first string 132 is configured to be cut and removed or weaken by degradation to detach the anchor portion 104 from the remainder of the stent 100. The second string 134 and the cord 128 may then function to at least partially collapse an end of the stent body 102 for ease of removal, as shown in FIG. 9.

FIG. 9 shows an intentional force being applied to the second string 134 and/or the cord 128 by way of a grasping strand 129. The grasping strand 129 may include an optional handle 131, as shown. The force may be transferred to the crown 126 of the stent body 102 through the second string 134 (or another string). Particularly when the flange 122, the crown 126, and/or other end structure of the stent body 102 is relatively compliant, such a tension may provide a drawstring effect, thus pulling the diameter of the end of the stent body 102 inwards towards a central point along the stent's longitudinal axis. This collapsing feature may be limited to the end of the stent body 102 (e.g., the crown or flange), or it may extend along the central portion of the stent body 102. Thus, at least one location of the stent body 102, and preferably a location with a maximum diameter when in a default state, decreases in diameter in a collapsed state initiated by tension in the cord 128 and the second string 134. Advantageously, this decrease in diameter/size may facilitate removal of the stent body 102 from the patient body without requiring an invasive removal procedure. A similar feature is described in U.S. Patent No. 9,271,854, filed as U.S. Patent Application No. 13/016,421 on January 28, 2011.

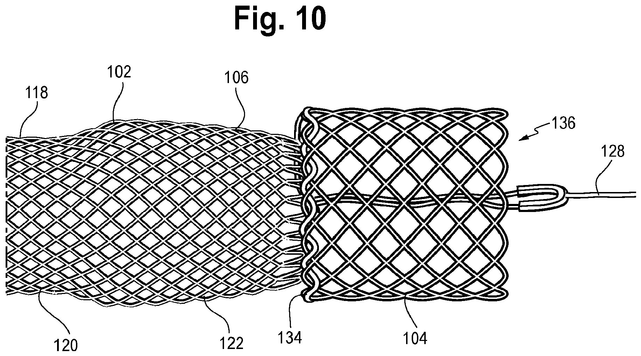

To further illustrate, FIG. 10 shows the proximal flange 122 in a collapsed state after the stent body 102 has been separated from the anchor portion 104. As shown, the cord 128 may extend through the lumen 136 of the anchor portion 104 when being tensioned by a medical professional and/or a medical device that engages the cord 128. The outer diameter of the flange 122 may collapse to the extent that it can freely move through the lumen 136 of the anchor portion 104. The diameter 118 of the central portion 120 of the stent body can also collapse to some extent, or it may simply be small enough to begin with where it does not inhibit removal of the stent body 102 through the lumen 136 of the anchor portion 104.

While not shown, more than one string may be included to provide a collapsing effect at more than one location. For example, the proximal end 106 and the distal end 108 (FIG. 1) of the stent body 102 may each be associated with a string that acts as a drawstring when pulled, and/or additional strings may be placed along the length of the stent body 102 between the distal end 108 (FIG. 1) and the proximal end 106. Further, it is contemplated that only one strand (e.g., the second string 134 or the cord 128) may be necessary to provide the drawstring effect. Additionally, it is contemplated that one pull of one cord or string (e.g., a pull of the cord 128) may simultaneously accomplish both of (1) collapsing a diameter of a stent body, and also (2) separation of one portion of the stent from another (e.g., releasing the anchor portion 104 from the stent body 102).

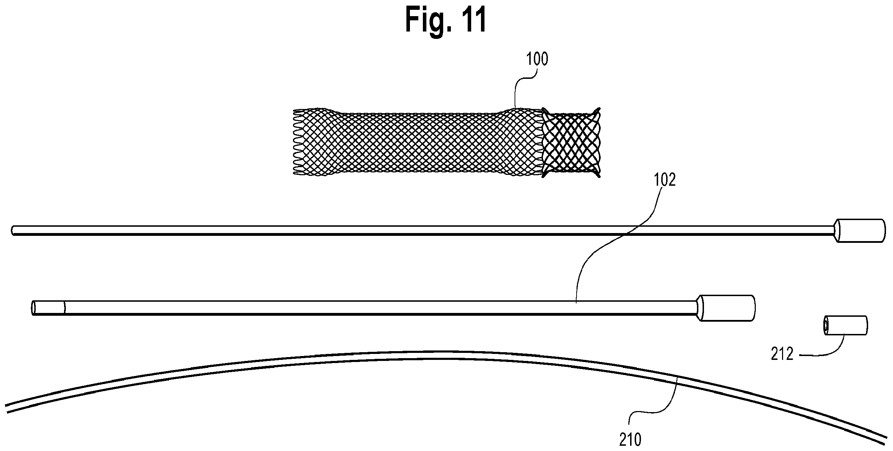

As shown in FIG. 11, a delivery system 200 may be provided to facilitate deployment of the stent 100. Without limitation, the delivery system 200 may include a sheath 202 for constricting the stent 100 during deployment to a target area in the body, a plunger 208 to press the stent 100 into the sheath 202 prior to deployment, an inner tube 210 that may extend through the sheath 202 and stent 100 to guide the device during deployment and/or provide fluid communication to a body area from a proximal location, and an adaptor 212 (where the adapter may be a luer-lock adapter used to facilitate attachment to a syringe to facilitate fluid passage). One example of a stent delivery system is the system provided with an Evolution^{®} Esophageal Controlled-Release Stent (commercialized by Cook^{®} Medical). The stent 100 is optimally packaged with the delivery system 200 during manufacturing or otherwise prior to delivery to a medical facility, but alternatively a medical professional may place the stent 100 in the delivery system 200 on-site prior to a medical procedure.

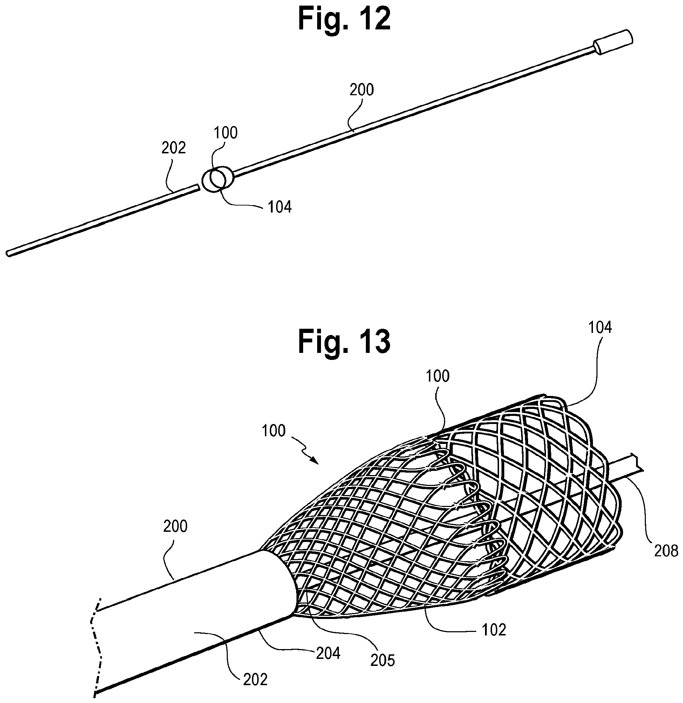

FIG. 12 shows the stent 100 and delivery system 200 in a storage state. As depicted, the sheath 202 surrounds the majority of the stent 100, and all of (or nearly all of) the stent body 102 (not shown) is in a constrained state within the sheath 202. The anchor portion 104 is depicted as being located at least partially outside the sheath 202 in an unconstrained state. This condition may be suitable for packaging and storage where the anchor portion 104 is not formed of a material with shape-recovery characteristics capable of retaining a proper "deployed" or default shape and state after remaining constrained within the sheath for a long time period (e.g., days, weeks, months, or even years). For example, while the stent body 102 may be formed of a nitinol or another suitable material with a high degree of shape-memory, the anchor portion 104 may formed of a different material selected primarily for its bioabsorbability rather than its mechanical characteristics, and therefore it may lack sufficient shape-memory to remain within the sheath 202 for days or weeks (or longer) without permanently changing its default shape due to plastic deformation. However, by leaving the anchor portion 104 in a default (substantially non-constrained) state during packaging and storage, its shape characteristics will be retained.

Referring to FIG. 13, the delivery system 200 may include a funnel 205 located at a proximal end 204 to assist loading the proximal end of the stent 100, particularly the anchor portion 104, to within the sheath 202. As shown, the funnel 205 is located adjacent the proximal end of the stent body 102, and the proximal end of the stent body 102 expands in diameter as it extends away from the funnel 205 until reaching the anchor portion 104, which is in a default (substantially non-constrained) state. At a selected time period before the stent 100 is to be deployed (e.g., minutes, hours, or even several days prior to deployment), the proximal end 106 of the stent body 102 along with the anchor portion 104 may be moved to at least partially into the sheath 202 by operating a plunger 208, which typically includes a pusher (e.g., a friction sleeve) that forces the stent 100 into the sheath 202 when moved distally with respect to the sheath 202. Since the stent 100 is already partially pre-loaded into the sheath 202, and since loading the remainder of the stent requires only a simple motion of the plunger 208, the remainder of the loading can easily and efficiently be accomplished by a medical professional at a medical facility rather than prior to on-site delivery.

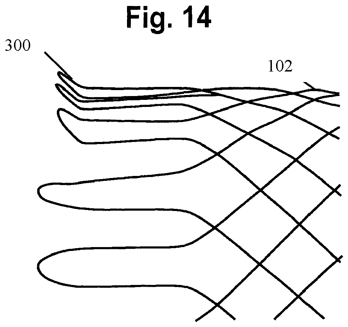

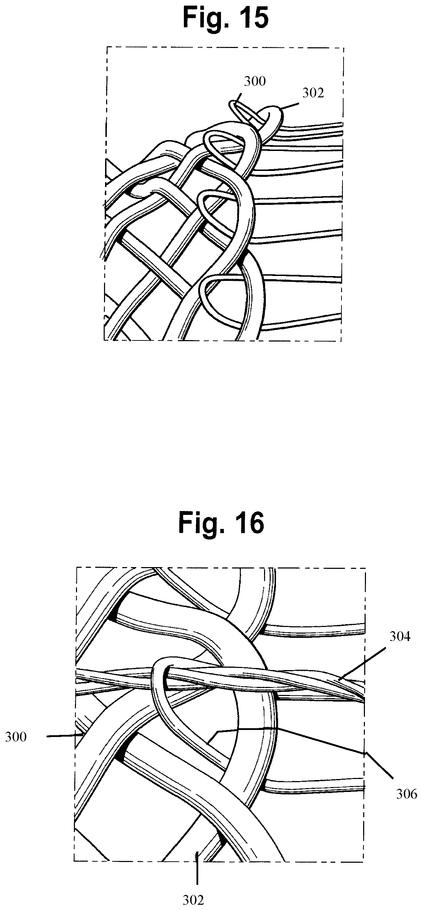

Referring now to FIGS. 14-18B, in addition to the examples described above (or as an alternative), any other suitable removable/releasable attachment mechanism may be additionally or alternatively included for separating the stent body 102 from the anchor portion 104. Referring to FIGS. 14-15, it is contemplated that a crown tip 300 of at least one of the stent body 102 and the anchor portion 104 may be bent outward. As shown in FIG. 15, the bend may case the end of the crown tip 300 to extend through an opening 302 in the anchor portion 104 (and, notably, the bent crown tip may alternatively be included as a portion of the anchor portion 104). As shown in FIG. 16, a pin 304 may be inserted through the opening 306 defined by the end of the crown tip 300 to retain the end of the crown tip 300 in the opening 302. The pin 304 may alternatively be a string (e.g., a drawstring) or another feature. In certain embodiments, for example, pulling a drawstring (such as the grasped strand 129 shown in FIG. 9) may have a dual purposes: (1) removing the pin 304 and/or otherwise detaching the anchor portion 104 from the stent body 102, and (2) causing the stent body 102 to enter a collapsed state.

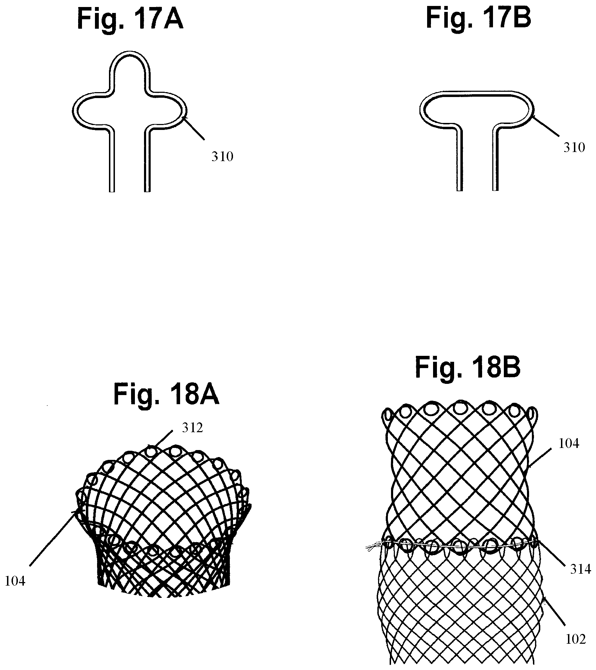

In some embodiments, the anchor portion 104 may be secured to the stent portion 102 without the use of a string or pin. For example, in some embodiments, the crown tip 300 may have one or more wings 310 (shown in two of the many possible orientations in FIGS. 17A-17B). The crown tip 300 may be relatively compliant such that the wings 310 deform as the crown tip 300 is placed into an opening of another component (e.g., the opening 306 shown in FIG. 15) such that the crown tips 300 are secured via clipping action. It is contemplated that the bend orientation of the crown tips 300 (as shown in FIG. 14) may be sufficient for radial clipping even if wings are not included, but the wings 310 may provide enhancement.

Another embodiment is shown in FIGS. 18A-18B. As shown, the anchor portion 104 includes a set of eyelets 312. Additionally or alternatively, the stent portion 102 may include similar or identical eyelets. The eyelets 312 may be formed integrally with the remainder of the anchor portion 104 (e.g., by twisting the wire or other material forming the anchor portion 104). As shown, a cord 314 may extend through the eyelets 312 (and also through openings in the stent body 102) to hold the components together. If and when detachment is desirable, the cord 314 may be removed with any suitable process (e.g., via one or more of the methods described above). For example (which is not limiting), the cord may be formed with a biodegradable material that dissolves over time.

While not shown, it is further contemplated that a lockstitch may be used (e.g., such that one pull of a string releases the components from one another).

The figures and disclosure are intended to be illustrative and not exhaustive. This description will suggest many variations and alternatives to one of ordinary skill in the art.

## Claims

1. A stent (100) comprising:
a tubular stent body (102) having a lumen extending therethrough; and
an anchor portion (104) attached to an end of the tubular stent body, wherein the lumen continues through the anchor portion (104), and wherein the anchor portion includes at least one opening configured to facilitate tissue ingrowth,
wherein the anchor portion (104) is detachable from the tubular stent body (102) while the stent is in a deployed state,
the stent further comprising at least one strand (128, 132, 134) securing the end of the tubular stent body to the anchor portion wherein the at least one strand (128, 132, 134) is at least partially formed of a bioabsorbable material such that the at least one strand weakens over time when the stent is deployed in a patient body,
wherein the anchor portion (104) includes a bioabsorbable material configured to degrade at a predetermined rate, and the at least one strand (128, 132, 134) is configured to degrade faster than the material of the anchor portion (104).

2. The stent of claim 1, wherein the at least one strand is configured to disengage from at least one of the tubular stent body (102) and the anchor portion (104) to detach the anchor portion from the tubular stent body.

3. The stent of any of claims 1 to 2, further comprising a strand (128, 134) secured to the end of the tubular stent body (102), wherein the strand is configured to collapse the end of the tubular stent body upon receipt of a tension force when the tubular stent body (102) and the anchor portion (104) are detached.

4. The stent of any of claims 1 to 3, wherein the tubular stent body (102) includes a covering to prevent tissue ingrowth.

5. The stent of any of claims 1 to 4, wherein the end of the tubular stent body includes a flange (122), and wherein the flange has a diameter that is larger than a diameter of a central portion of the tubular stent body (102).

6. The stent of claim 5, wherein a diameter of the anchor portion (104) is larger than the diameter of the central portion of the tubular stent body (102).

7. The stent of any of claims 1 to 6, further comprising a second removable stent portion coupled to a second end of the tubular stent body.

## Patentansprüche

1. Stent (100), umfassend:
einen rohrförmigen Stentkörper (102) mit einem sich dort hindurch erstreckenden Lumen und
einen Ankerabschnitt (104), der an einem Ende des rohrförmigen Stentkörpers angebracht ist,
wobei das Lumen durch den Ankerabschnitt (104) weitergeht und wobei der Ankerabschnitt mindestens eine Öffnung aufweist, die zur Ermöglichung des Hineinwachsens von Gewebe ausgestaltet ist,
wobei der Ankerabschnitt (104) von dem rohrförmigen Stentkörper (102) ablösbar ist, während der Stent in einem ausgebrachten Zustand ist,
wobei der Stent ferner mindestens einen Strang (128, 132, 134) umfasst, der das Ende des rohrförmigen Stentkörpers an dem Ankerabschnitt befestigt, wobei der mindestens eine Strang (128, 132, 134) mindestens zum Teil aus einem biologisch absorbierbaren Material ausgebildet ist, so dass der mindestens eine Strang im Laufe der Zeit geschwächt wird, wenn der Stent in dem Körper eines Patienten ausgebracht ist,
wobei der Ankerabschnitt (104) ein biologisch absorbierbares Material aufweist, das zum Abbau mit einer vorbestimmten Rate ausgestaltet ist, und der mindestens eine Strang (128, 132, 134) ist zu einem schnelleren Abbau als das Material des Ankerabschnitts (104) ausgestaltet.

2. Stent nach Anspruch 1, wobei der mindestens eine Strang dazu ausgestaltet ist, aus dem rohrförmigen Stentkörper (102) und/oder dem Ankerabschnitt (104) auszurücken, um den Ankerabschnitt von dem rohrförmigen Stentkörper zu lösen.

3. Stent nach einem der Ansprüche 1 bis 2, ferner umfassend einen Strang (128, 134), der an dem Ende des rohrförmigen Stentkörpers (102) befestigt ist, wobei der Strang dazu ausgestaltet ist, das Ende des rohrförmigen Stentkörpers bei Aufnahme einer Zugkraft zusammenfallen zu lassen, wenn der rohrförmige Stentkörper (102) und der Ankerabschnitt (104) abgelöst werden.

4. Stent nach einem der Ansprüche 1 bis 3, wobei der rohrförmige Stentkörper (102) eine Abdeckung aufweist, um zu verhindern, dass Gewebe einwächst.

5. Stent nach einem der Ansprüche 1 bis 4, wobei das Ende des rohrförmigen Stentkörpers einen Flansch (122) aufweist und wobei der Flansch einen Durchmesser hat, der größer als ein Durchmesser eines mittleren Abschnitts des rohrförmigen Stentkörpers (102) ist.

6. Stent nach Anspruch 5, wobei ein Durchmesser des Ankerabschnitts (104) größer als der Durchmesser des mittleren Abschnitts des rohrförmigen Stentkörpers (102) ist.

7. Stent nach einem der Ansprüche 1 bis 6, ferner umfassend einen zweiten entfernbaren Stentabschnitt, der an ein zweites Ende des rohrförmigen Stentkörpers gekoppelt ist.

## Revendications

1. Endoprothèse (100) comprenant:
un corps d'endoprothèse tubulaire (102) présentant une lumière s'étendant à travers celui-ci; et
une partie ancrage (104) fixée à une extrémité du corps d'endoprothèse tubulaire, dans laquelle la lumière continue à travers la partie ancrage (104), et dans laquelle la partie ancrage comporte au moins une ouverture conçue pour faciliter une croissance tissulaire,
dans laquelle la partie ancrage (104) est détachable du corps d'endoprothèse tubulaire (102) pendant que l'endoprothèse est dans un état déployé,
l'endoprothèse comprenant en outre au moins un brin (128, 132, 134) solidarisant l'extrémité du corps d'endoprothèse tubulaire à la partie ancrage dans laquelle l'au moins un brin (128, 132, 134) est au moins partiellement formé d'un matériau biorésorbable de telle sorte que l'au moins un brin s'affaiblisse au fil du temps lorsque l'endoprothèse est déployée dans le corps d'un patient,
dans laquelle la partie ancrage (104) comporte un matériau biorésorbable conçu pour se dégrader à une vitesse prédéterminée, et l'au moins un brin (128, 132, 134) est conçu pour se dégrader plus rapidement que le matériau de la partie ancrage (104).

2. Endoprothèse selon la revendication 1, dans laquelle l'au moins un brin est conçu pour se désolidariser d'au moins l'un du corps d'endoprothèse tubulaire (102) et de la partie ancrage (104) pour détacher la partie ancrage du corps d'endoprothèse tubulaire.

3. Endoprothèse selon l'une quelconque des revendications 1 à 2, comprenant en outre un brin (128, 134) fixé à l'extrémité du corps d'endoprothèse tubulaire (102), dans laquelle le brin est conçu pour aplatir l'extrémité du corps d'endoprothèse tubulaire à la réception d'une force de tension lorsque le corps d'endoprothèse tubulaire (102) et la partie ancrage (104) sont détachés.

4. Endoprothèse selon l'une quelconque des revendications 1 à 3, dans laquelle le corps d'endoprothèse tubulaire (102) comporte un revêtement pour empêcher la croissance tissulaire.

5. Endoprothèse selon l'une quelconque des revendications 1 à 4, dans laquelle l'extrémité du corps d'endoprothèse tubulaire comporte une bride (122), et dans laquelle la bride a un diamètre qui est plus grand qu'un diamètre d'une partie centrale du corps d'endoprothèse tubulaire (102).

6. Endoprothèse selon la revendication 5, dans laquelle un diamètre de la partie ancrage (104) est plus grand que le diamètre de la partie centrale du corps d'endoprothèse tubulaire (102).

7. Endoprothèse selon l'une quelconque des revendications 1 à 6, comprenant en outre une seconde partie d'endoprothèse amovible accouplée à une seconde extrémité du corps d'endoprothèse tubulaire.
